# EUROPEAN PATENT SPECIFICATION

(11) **EP 0 662 966 B1**
(45) Date of publication and mention of the grant of the patent: **24.05.2000**
(21) Application number: 93921839.2
(22) Date of filing: 23.09.1993
(51) Int. Cl.: C07D 309/28

(54) **SYNTHESIS OF N-ACETYL NEURAMINIC ACID DERIVATIVES**
HERSTELLUNG VON DERIVATEN VON A-ACETYL NEURAMINICSÄUREN
SYNTHESE DE DERIVES DE L'ACIDE N-ACETYLE NEURAMINIQUE

(30) Priority: 25.09.1992 GB 9220241
(43) Date of publication of application: 19.07.1995
(73) Proprietor: BIOTA SCIENTIFIC MANAGEMENT PTY. LTD., Melbourne, VIC 3004 (AU)
(72) Inventor: WEIR, Niall, Galbraith, Glaxo Group Research Ltd., Middlesex UB6 0HE (GB); CHANDLER, Malcolm, Glaxo Group Research Ltd., Middlesex UB6 0HE (GB); BAMFORD, Mark, James, Glaxo Group Research Ltd., Middlesex UB6 0HE (GB)
(74) Representative: Woodman, Derek
(86) International application number: EP9302574
(87) International publication number: WO9407885

(56) References cited:
- EP-A- 0 539 204
- WO-A-91/16320
- WO-A-93/12105
- LIEBIGS ANNALEN DER CHEMIE vol. 1991, no. 2, February 1991, WEINHEIM, pages 129 - 134 ERWIN SCHREINER ET. AL.
- HOUBEN-WEYL vol. E4, 01 January 1983, page 609 HAGEMANN, H. 'Methoden der Organischen Chemie'
- CARBOHYDRATE RESEARCH vol.194, 1989,Amsterdam NL pages C15-C18, ZBIRAL E. ET AL "Synthesis of the 4-acetamido-4-deoxy analogue of N-acetylneuraminic acid and its behaviour towards CMP-sialate Synthase"

## Description

The present invention relates to a process for the preparation of derivatives of N-acetyl neuraminic acid. More particularly the invention relates to a process for the preparation of 5-acetamido-2,3,4,5-tetradeoxy-4-guanidino-D-glycero-D-galacto-non-2-enopyranosonic acid (the 4-guanidino analogue of DANA; also known as 5-(acetylamino)-2,6 anhydro-3,4,5-trideoxy-4-guanidino-D-glycero-D-galacto-non-2-enonic acid) and derivatives thereof.

PCT/AU91/00161 (publication no. WO91/16320) describes a number of derivatives of 5-acetamido-2,3,5-trideoxy-D-glycero-D-galacto-non-2-enopyranasonic acid (2,3-dideoxy-2,3-didehydro-N-acetyl-neuraminic acid; DANA) including the 4-guanidino analogue of DANA. The 4-guanidino analogue of DANA is prepared by the reaction of the corresponding O-acyl protected 4-amino analogue of DANA by reaction with S-methylisothiourea followed by deprotection.

HOUBEN-WEYL, VOL.E4 (KOHLENSÄUREDERIVATE), 1983, PAGE 609 discloses the addition of amines to cyanoamides but not in the context of the synthesis of a complex, biologically active molecule, such as detailed herein.

We have now found an improved method of preparing the 4-guanidino analogue of DANA.

The invention thus provides in a first aspect a method for the preparation of 5-acetamido-2,3,4,5-tetradeoxy-4-guanidino-D-glycero-D-galacto-non-2-enopyranosonic acid which comprises amination of 5-acetamido-4-cyanoamide-2,3,4,5-tetradeoxy-D-glycero-D-galacto-non-2-enopyranosonic acid (the 4-cyanoamide analogue of DANA, also known as the 4-cyanamido analogue of DANA).

The structure of the 4-amino, 4-cyanoamide and 4-guanidino analogues of DANA are shown below:

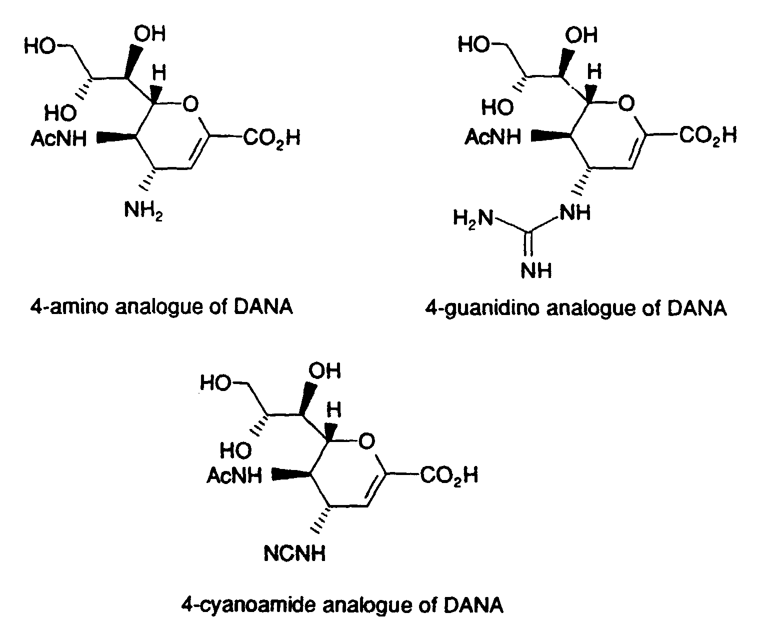

The amination may be effected by any suitable source of ammonia for example gaseous ammonia, liquid ammonia or aqueous ammonia. Preferably aqueous ammonia eg 0.880sg ammonia is used.

The reaction may be carried out at elevated temperature, for example from about 50° to 100°C. Conveniently the reaction is carried out at reflux when aqueous ammonia is employed.

The molar ratio of the 4-cyanoamide analogue of DANA to the source of ammonia employed in the reaction may be from about 1:50 to about 1:250 for example about 1:100.

A buffer is preferably employed in the reaction. Any suitable buffer may be employed for example ammonium salts such as ammonium formate or ammonium acetate and alkali metal salts - preferably the buffer is employed in a molar excess of about 5 to 7, for example 6, fold.

The desired 4-guanidino analogue of DANA may be isolated by any conventional method from the reaction mixture, for example by crystallisation or chromatography. In particular the 4-guanidino analogue of DANA may be isolated by treatment of an aqueous solution with an organic solvent in which the compound is insoluble. Such solvents include for example *iso*propyl alcohol (IPA) and acetone.

The 4-cyanoamide analogue of DANA may be prepared from the corresponding 4-amino analogue of DANA by reaction with a source of electrophilic cyanide. Preferably a cyanogen halide, for example cyanogen bromide, is employed as the source of cyanide.

The preparation of the 4-cyanoamide analogue of DANA is preferably carried out in a protic, non-aqueous solvent for example a lower aliphatic alcohol such as ethanol or, preferably, methanol conveniently in the presence of a buffer such as sodium acetate.

The reaction is conveniently carried out at ambient temperature.

The 4-cyanoamide analogue of DANA is a novel compound and forms a further aspect of the invention.

The 4-amino analogue of DANA is a known compound (International Application Publication No WO91/16320)

The present invention is further described by the following examples which are for illustrative purposes only and should not be construed as a limitation of the invention.

### Example 1

(i) 5-Acetamido-4-amino-2,3,4,5-tetradeoxy-D-glycero-D-galacto-non-2-enopyranosonic acid (3g,10.35mmol) was treated with cyanogen bromide (1.1 eq) in dry methanol in the presence of sodium acetate (2.2 eq) for 2 days at 21°C . The reaction mixture was then treated with methanol: propan-2-ol (1:1 260ml) to precipitate out the desired 5-acetamido-4-cyanoamide-2,3,4,5-tetradeoxy-D-glycero-D-galacto-non-2-enopyranosonic acid.
(ii) The product of step (i) (1 g, 3.17mmol) was reacted in the presence of ammonium formate (6 eq) with 0.880 sg ammonia (20ml) at 88°C for 4hrs. The product was purified by chromatography on ion-exchange resin (Dowex 50W x8 (H⁺)15ml, eluted with 0.6M triethylamine) followed by crystallisation from water/propan-2-ol (8:20) to give 5-acetamido-2,3,4,5-tetradeoxy-4-guanidino-D-glycero-D-galacto-non-2-enopyranosonic acid (220mg) identical with authentic product.

## Claims

1. A method for the preparation of 5-acetamido-2,3,4,5-tetradeoxy-4-guanidino-D-glycero-D-galacto-non-2-eno-pyranosonic acid which comprises amination of 5-acetamido-4-cyanoamide-2,3,4,5-tetradeoxy-D-glycero-D-galacto-non-2-enopyranosonic acid.

2. A method as claimed in claim 1 wherein the amination is effected with gaseous ammonia, liquid ammonia or aqueous ammonia.

3. A method as claimed in claim 1 or claim 2 wherein the amination is effected with aqueous ammonia.

4. A method as claimed in any one of claims 1 to 3 wherein the amination is effected with 0.880 sg ammonia.

5. A method as claimed in any one of claims 1 to 4 wherein the molar ratio of 5-acetamido-4-cyanoamide-2,3,4,5-tetradeoxy-D-glycero-D-galacto-non-2-enopyranosonic acid to ammonia is from about 1:50 to 1:250.

6. A method as claimed in any one of claims 1 to 5 wherein the molar ratio of 5-acetamido-4-cyanoamide-2,3,4,5-tetradeoxy-D-glycero-D-galacto-non-2-enopyranosonic acid to ammonia is about 1:100.

7. A method as claimed in any one of claims 1 to 6 wherein the reaction is carried out at a temperature of from about 50-100°C.

8. A method as claimed in any one of claims 1 to 7 wherein the reaction is carried out in the presence of a buffer.

9. A method as claimed in any one of claims 1 to 8 wherein the reaction is carried out in the presence of a buffer selected from an ammonium salt or an alkali metal salt.

10. 5-Acetamido-4-cyanoamide-2,3,4,5-tetradeoxy-D-glycero-D-galacto-non-2-enopyranosonic acid.

## Patentansprüche

1. Verfahren zur Herstellung von 5-Acetamido-2,3,4,5-tetradesoxy-4-guanidino-D-glycero-D-galacto-non-2-eno-pyranosesäure, umfassend die Aminierung von 5-Acetamido-4-cyanamid-2,3,4,5-tetradesoxy-D-glycero-D-galacto-non-2-enopyranosesäure.

2. Verfahren nach Anspruch 1, wobei die Aminierung mit gasförmigem Ammoniak, flüssigem Ammoniak oder wässrigem Ammoniak durchgeführt wird.

3. Verfahren nach Anspruch 1 oder 2, wobei die Aminierung mit wässrigem Ammoniak durchgeführt wird.

4. Verfahren nach einem der Ansprüche 1 bis 3, wobei die Aminierung mit 0,880 sg Ammoniak durchgeführt wird.

5. Verfahren nach einem der Ansprüche 1 bis 4, wobei das Molverhältnis von 5-Acetamido-4-cyanamid-2,3,4,5-tetradesoxy-D-glycero-D-galacto-non-2-enopyranosesäure zu Ammoniak etwa 1:50 bis 1:250 beträgt.

6. Verfahren nach einem der Ansprüche 1 bis 5, wobei das Molverhältnis von 5-Acetamido-4-cyanamid-2,3,4,5-tetradesoxy-D-glycero-D-galacto-non-2-enopyranosesäure zu Ammoniak etwa 1:100 beträgt.

7. Verfahren nach einem der Ansprüche 1 bis 6, wobei die Reaktion bei einer Temperatur von etwa 50-100°C durchgeführt wird.

8. Verfahren nach einem der Ansprüche 1 bis 7, wobei die Reaktion in Gegenwart eines Puffers durchgeführt wird.

9. Verfahren nach einem der Ansprüche 1 bis 8, wobei die Reaktion in Gegenwart eines Puffers, ausgewählt aus einem Ammoniumsalz oder einem Alkalimetallsalz, durchgeführt wird.

10. 5-Acetamido-4-cyanamid-2,3,4,5-tetradesoxy-D-glycero-D-galacto-non-2-enopyranosesäure.

## Revendications

1. Procédé de préparation de l'acide 5-acétamido-2,3,4,5-tétradésoxy-4-guanidino-D-glycéro-D-galacto-non-2-énopyrannosonique, qui comprend l'animation de l'acide 5-acétamido-4-cyanoamide-2,3,4,5-tétradésoxy-D-glycéro-D-galacto-non-2-énopyrannosonique.

2. Procédé selon la revendication 1, dans lequel l'amination est effectuée avec de l'ammoniac gazeux, de l'ammoniac liquide ou de l'ammoniaque.

3. Procédé selon la revendication 1 ou 2, dans lequel l'amination est effectuée avec de l'ammoniaque.

4. Procédé selon l'une quelconque des revendications 1 à 3, dans lequel l'amination est effectuée avec une ammoniaque ayant une densité de 0,880.

5. Procédé selon l'une quelconque des revendications de 1 à 4 dans lequel le rapport en moles de l'acide 5-acétamido-4-cyanoamide-2,3,4,5-tétradésoxy-D-glycéro-D-galacto-non-2-énopyrannosonique à l'ammoniac est d'environ 1:50 à 1:250.

6. Procédé selon l'une quelconque des revendications 1 à 5, dans lequel le rapport en moles de l'acide 5-acétamido-4-cyanoamide-2,3,4,5-tétradésoxy-D-glycéro-D-galacto-non-2-énopyrannosonique à l'ammoniac est d'environ 1:100.

7. Procédé selon l'une quelconque des revendications 1 à 6, dans lequel la réaction est mise en oeuvre à une température d'environ 50 à 100°C.

8. Procédé selon l'une quelconque des revendications 1 à 7, dans lequel la réaction est mise en oeuvre en présence d'un tampon.

9. Procédé selon l'une quelconque des revendications 1 à 8, dans lequel la réaction est mise en oeuvre en présence d'un tampon choisi parmi les sels d'ammonium et les sels de métaux alcalin.

10. Acide 5-acétamido-4-cyanoamide-2,3,4,5-tétradésoxy-D-glycéro-D-galacto-non-2-énopyrannosonique.
